# EUROPEAN PATENT SPECIFICATION

(11) **EP 1 824 501 B1**
(45) Date of publication and mention of the grant of the patent: **19.08.2009**
(21) Application number: 05804084.1
(22) Date of filing: 15.11.2005
(51) Int. Cl.: A61K 38/00, A61P 3/10, A23L 1/305, C07K 5/103, C07K 14/47

(54) **PROTEIN HYDROLYSATE WITH ANTIDIABETIC EFFECT**
PROTEINHYDROLYSAT MIT ANTIDIABETISCHER WIRKUNG
HYDROLYSAT DE PROTEINES A EFFET ANTIDIABETIQUE

(30) Priority: 15.11.2004 US 627843 P
(43) Date of publication of application: 29.08.2007
(73) Proprietor: MG PHARMA INC., Osaka 567-0085 (JP)
(72) Inventor: KAGAWA, Kyoichi, 5670046 (JP); NARUSE, Toshihiko, 2740822 (JP); FUKUHAMA, Chizuko, 6660033 (JP)
(74) Representative: Müller-Boré & Partner Patentanwälte
(86) International application number: PCT/JP2005/021318
(87) International publication number: WO 2006/052031

(56) References cited:
- EP-A- 0 044 032
- EP-A- 0 420 979
- EP-A- 0 753 526
- WO-A1-89/06970
- WO-A1-94/21671
- JP-A- 02 154 693
- JP-A- 05 344 863
- STEINER G, VRANIC M: "Hyperinsulinemia and hypertriglyceridemia, a vicious cycle with atherogenic potential." INT J OBES, vol. 6, no. suppl., 1982, pages 117-124, XP002466930
- STEINER G: "Hypertriglyceridemia and carbohydrate intolerance: interrelations and therapeutic implications." AM J CARDIOL, vol. 57, no. 14, 1986, pages 27G-30G, XP002466931
- KAGAWA K ET AL: 'GLOBIN DIGEST, ACIDIC PROTEASE HYDROLYSATE, INHIBITS DIETARY HYPERTRIGLYCERIDEMIA AND VAL-VAL-TYR-PRO, ONE OF ITS CONSTITUENTS, POSSESSES MOST SUPERIOR EFFECT.' LIFE SCIENCES. vol. 58, no. 20, 1996, pages 1745 - 1755, XP000983458
- KAGAWA K ET AL: 'Supressive Effect of Globin Digest on Postprandial Hyperlipidemia in Male Volunteers.' THE JOURNAL OF NUTRITION. vol. 128, no. 1, 1998, pages 56 - 60, XP002995984

## Description

The present invention relates to uses of a peptide having the sequence Val-Val-Typ-Pro (SEQ ID NO:1) or a globin hydrolysate containing the peptide that exhibit an action of preventing or ameliorating hyperglycemic conditions (an action of inhibiting an increase in blood sugar) through induction of insulin secretion or enhancement of insulin sensitivity by increasing insulin receptors in a diabetic subject or a subject in a preclinical stage (borderline diabetes). Furthermore, the present invention relates to a peptide having the sequence Val-Val-Typ-Pro (SEQ ID NO:1) or a globin hydrolysate containing the peptide for use in preventing or treating diseases resulting from hyperglycemia, in particular, diabetes and diabetic complications. In this specification, such compositions are sometimes collectively called antidiabetic compositions.

The blood-sugar level in the living body is controlled by the balance between the hypoglycemic action of insulin and the blood-sugar-increasing actions of adrenalin, glucagon, glucocorticoid and the like. In particular, insulin inhibits glycogenolysis and gluconeogenesis in the liver to hinder glucose production and reduce the amount of glucose released from the liver into the blood and, at the same time, insulin increases glucose uptake into skeletal muscles and white adipose tissues, thereby lowering the blood sugar level. In contrast, adrenalin, glucagon, etc., prompt glycogenolysis and gluconeogenesis in the liver and enhance glucose release therefrom, thereby increasing the blood sugar level.

Diabetes is a metabolic disease in which a hyperglycemic state persists due to an acute or chronical decrease in the action of insulin, resulting in disorders in sugar metabolism, lipid metabolism, amino acid metabolism, etc.

Diabetes is categorized as either insulin dependent or non-insulin dependent. Dietary therapy and oral hypoglycemic agents are not effective in treating insulin-dependent diabetes, and insulin-dependent diabetes is treatable only by insulin because insulin secretory capacity is reduced or absent. In contrast, with respect to non-insulin-dependent diabetes, which accounts for 90 percent of diabetic patients, although the action of insulin is weakened compared with that in normal people, treatment thereof does not necessarily require insulin. Alimentary therapy and exercise therapy are usually performed, and if they are not sufficient, chemotherapy by hypoglycemic agents is then used concomitantly.

As described above, diabetes is a disease resulting in metabolic disorders due to a persistent hyperglycemic condition, and it is a troublesome disease that may accompany many complications in the eyes, kidneys, nervous system, cardiovascular system, skin, etc. Such complications are generally considered to diminish when the blood sugar level is controlled to near normal levels (Saishin Igaku Daijiten, 1988, p. 1211, Ishiyaku Publishers Inc., Japan).

Known pharmaceutical preparations for ameliorating hyperglycemic conditions include insulin preparations, sulfonyl urea preparations, biguanide preparations, insulin resistance improving preparations, *a*-glucosidase inhibitors, etc. Insulin preparations are therapeutic agents for insulin-dependent diabetes and while they reliably lower blood sugar levels they carry the risk of causing hypoglycemia. Sulfonyl urea preparations are drugs that lower blood sugar levels by enhancing endogenous insulin secretion by stimulating pancreatic B-cells. They may cause hypoglycemia as a side effect due to insulin secretion induced irrespective of blood sugar levels. Biguanide preparations are drugs that lower blood sugar levels by inhibiting gluconeogenesis in the liver, increasing sugar consumption in the skeletal muscles and the like, and inhibiting intestinal absorption of sugar, and are advantageous in not causing hypoglycemia in either normal people or diabetic patients. However, biguanide preparations are problematic in often resulting in comparatively severe lactic acidosis. Insulin resistance improving preparations (e.g., thiazolidine derivatives and the like) are drugs that lower blood sugar levels by fortifying the action of insulin and activating insulin receptor kinases. It has been pointed out, however, that digestive symptoms, edema, etc., develop as side effects, and the amounts of red blood cells, hematocrit and hemoglobin are decreased and the amount of LDH is increased (Atarashii Tonyobyo Chiryoyaku (New Diabetic Medicines), pp. 90-99, 1994, Iyaku (Medicine and Drug) Journal Co., Ltd., Japan). a-Glucosidase inhibitors exhibit an action of inhibiting an increase in after-meal blood sugar levels by retarding the digestion and absorption of sugars in the gastrointestinal tract, but are problematic in terms of side effects such as bloating feeling, borborygmus, diarrhea, etc (Joslin's Diabetes mellitus, 13th ed., pp. 521-522).

It can thus be said that methods for effectively treating or preventing diabetes and complications thereof have not yet been sufficiently established.

Further, Kagawa et al. (The Journal of Nutrition, vol. 128, no. 1, pages 56-60, 1998) describes the effect of globin digest on far absorption and catabolism in humans, Kagawa et al. (Life Sciences, vol. 58, no. 20, pages 1745-1755, 1996) describes the effect of globin digest on serum triglyceride levels and the excretion of administered lipids, WO-A1-9421671 discloses a peptide having an adipocyte differentiation inhibitor function, as well as a health food and a feed containing said peptide, WO-A1-8906970 discloses a lipid metabolism improving agent for humans or animals, as well as a method of its use, Steiner and Vranic (International Journal of Obesity, vol. 6, pages 117-124, 1986) describe the physiological consequences of hypertriglyceridemia, and Steiner (American Journal of Cardiology, vol. 57, no. 14, pages 27G-30G, 1986) describes the relationship between hypertriglyceridemia and carbohydrate intolerance, as well as the consequences thereof.
Figure 1 shows a gel filtration chromatogram of a globin proteolysate (Preparation Example 1).
Figure 2 shows the result (chromatogram) of reverse phase (acid) chromatography performed in Preparation Example 2(3).
Figure 3 shows the result (chromatogram) of reverse phase (neutral) chromatography performed in Preparation Example 2(4).

An object of the present invention is to provide uses of a peptide having the sequence Val-Val-Typ-Pro (SEQ ID NO:1) or a globin hydrolysate containing the peptide for preparing an antidiabetic agent, a food composition and feed composition that have an action of enhancing insulin secretion or an action of inhibiting an increase in blood sugar. Another object of the present invention is to provide a peptide having the sequence Val-Val-Typ-Pro (SEQ ID NO:1) or a globin hydrolysate containing the peptide for use in treatment or prevention of diabetes or complications thereof.

The inventors conducted extensive research to achieve the objects described above, and found that when a globin proteolysate is administered to mice with artificially-induced hyperglycemia, their blood sugar levels are significantly lowered and hyperglycemia is ameliorated. Moreover, the inventors ascertained that the active ingredient of the globin proteolysate is a peptide (VVYP) contained therein, and the action of inhibiting an increase in blood sugar is attributable to its insulin secretion enhancing action.

Furthermore, the inventors ascertained that the action of inhibiting an increase in a blood sugar level and action of enhancing insulin secretion of the globin proteolysate or the peptide (VVYP) are not observed in subjects having normal blood sugar levels, and these actions are specifically observed in diabetic patients and borderline diabetic patients who are at a preclinical stage.

Based on these findings, the inventors confirmed that the globin proteolysate or the peptide (VVYP) inhibits and ameliorates hyperglycemia (lowers a blood sugar level that is considered hyperglycemic), and is of use in preventing or treating diseases resulting from hyperglycemia, such as diabetes and complications thereof. The present invention was accomplished based on these findings.

In particular, the present invention includes the following embodiments.
1. Use of a peptide having the sequence Val-Val-Tyr-Pro (SEQ-ID NO:1) or a globin proteolysate containing the peptide for preparing a food composition for preventing or treating disease resulting from hyperglycemia.
2. Use of a peptide having the sequence Val-Val-Tyr-Pro (SEQ-ID NO:1) or a globin proteolysate containing the peptide for preparing a feed composition for preventing or treating disease resulting from hyperglycemia.
3. Use of a peptide having the sequence Val-Val-Tyr-Pro (SEQ-ID NO:1) or a globin proteolysate containing the peptide for preparing an antidiabetic agent.
4. Use of a peptide having the sequence Val-Val-Tyr-Pro (SEQ-ID NO:1) or a globin proteolysate containing the peptide for preparing a food for specified health use that has a function of inhibiting an increase in blood sugar or a function of enhancing insulin secretion and is used with an eye to such a function.
5. Use of a peptide having the sequence Val-Val-Tyr-Pro (SEQ-ID NO:1) or a globin proteolysate containing the peptide for preparing a feed for specified health use that has a function of inhibiting an increase in blood sugar or a function of enhancing insulin secretion and is used with an eye to such a function.
6. A peptide having the sequence Val-Val-Tyr-Pro (SEQ-ID NO:1) or a globin proteolysate containing the peptide for use in the prevention or treatment of a disease resulting from hyperglycemic condition caused by the weakening or lack of the action of insulin.
7. A peptide having the sequence Val-Val-Tyr-Pro (SEQ-ID NO:1) or a globin proteolysate containing the peptide for use in the prevention or treatment of a disease resulting from hyperglycemic condition caused by the weakening or lack of the action of insulin according to item 6, wherein the disease is diabetes or a diabetic complication.

A feature of the pharmaceutical composition, food composition, and feed composition is containing a globin proteolysate or a peptide (VVYP) as an active ingredient to be used in accordance with the present invention.

### (1) Pharmaceutical composition

Blood sugar increase inhibitors, insulin secretion enhancers, and compositions for preventing or treating diseases resulting from hyperglycemia are encompassed within the pharmaceutical compositions. A feature of such blood sugar increase inhibitors is containing a globin proteolysate or a peptide (VVYP) in an amount effective for preventing or ameliorating hyperglycemia (inhibiting an increase in blood sugar) of a diabetic patient or of a preclinical diabetic patient, i.e., borderline diabetic patient. A feature of such insulin secretion enhancers is containing a globin proteolysate or a peptide (VVYP) in an amount effective for enhancing insulin secretion in a diabetic patient or a borderline diabetic patient. Furthermore, such compositions for preventing and treating diseases resulting from hyperglycemia contain a globin proteolysate or a peptide (VVYP) in an amount effective for exhibiting a blood sugar increase inhibitory action or an insulin secretion enhancing action.

A globin proteolysate, i.e., one form of active ingredient of the pharmaceutical composition, is a hydrolyzate of a globin protein such as hemoglobin, myoglobin, etc. The kind of animals that can be a source of globin protein is not limited and a wide variety of animals such as bovines, pigs, sheep, humans, horses, etc., can be used.

Livestock meat, fish meat and the like containing large amounts of myoglobin can be used as globin protein sources.

The hydrolysis of globin protein and other procedures can be performed according to the methods described in WO89/06970. Hydrolysis is usually performed using one or more hydrolyzing enzymes such as acid proteases, neutral proteases, and alkaline proteases.

A specific example of a method for hydrolyzing a globin protein is dispersing a globin protein-containing material in water in an amount of 5 to 30 wt.% (solids content), adjusting the pH with an acid or base to be optimum for a protease, adding a protease in a single batch or in portions, and reacting the enzyme at 20 to 70°C for 3 to 48 hours.

The proteolysate thus obtained is used as the globin proteolysate having an action of inhibiting an increase in blood sugar or action of enhancing insulin secretion of the present invention. It may be used as is, used after drying, or used after adding thereto an extender such as carboxymethylcellulose, dextrin or the like followed by drying/solidifying.

A peptide (VVYP), i.e., another form of active ingredient of the pharmaceutical composition of the present invention, is contained in the aforementioned globin proteolysate in a proportion of about 1 wt.%, and can therefore be isolated/purified from the aforementioned globin proteolysate. Isolation or purification of the peptide (WYP) can be performed using, as starting materials, hydrolyzates of, in addition to globin proteins, for example, fish meat proteins, fish powders, and like animal proteins; corn proteins (zein), soy proteins, and like vegetable proteins.

The isolation or purification method described herein can be performed in the same manner as in known purification methods for proteins and peptides. For example, a fraction containing the peptide (WYP) can be obtained and then the peptide (VVW) can be isolated from the fraction by the use of salting-out, dialysis, ion-exchange resin, ultrafiltration, reverse phase chromatography or the like, or by the use of such methods in a suitable combination, if necessary. With respect to reverse phase chromatography mentioned among such purification methods, it is preferable that reverse phase chromatography under acidic conditions and reverse phase chromatography under neutral conditions are performed in combination.

The protein content of the fraction can be measured according to known protein determinations such as ninhydrin methods. The amino acid sequence of a peptide contained in the isolated fraction can be identified by known methods (amino acid analyses). The presence of the target peptide of the invention composed of the Val-Val-Tyr-Pro amino acid sequence can be verified thereby. In addition to the peptide (VVYP) isolated or purified according to an aforementioned method, a fraction containing it can be used as an active ingredient of the pharmaceutical composition of the present invention insofar as the fraction exhibits an action of inhibiting an increase in blood sugar or an action of enhancing insulin secretion.

The peptide (VVYP) can be chemically synthesized according to known peptide synthesis methods. Examples of peptide synthesis methods are azide methods, acid chloride methods, acid anhydride methods, mixed acid anhydride methods, DCC methods, activated ester methods, carboimidazole methods, redox methods, DCC-additive (HOMB, HOBt, HOSu) methods (Schreder, Luhke, The Peptide, volume 1, 1966, Academic Press, New York, USA; Izumiya et al., Peptide synthesis, Maruzen Co., Ltd. (1975); etc.), and like methods. Such peptide synthesis methods include solid phase and liquid phase synthesis methods.

In connection with such peptide synthesis methods, for amino acids having side-chain functional groups, e.g., tyrosine and threonine, their side chain functional groups are preferably protected. Known protecting groups are usable herein, for example, the benzyloxycarbonyl group (Cbz-), *t-*butoxycarbonyl group (Boc-), benzyl group (Bz-), etc. Such protecting groups can be removed according to known methods during the production of the peptide of the present invention.

The pharmaceutical composition may be composed entirely of the globin proteolysate or the peptide (VVYP). When the pharmaceutical composition is prepared in combination with other substances, the amount of the globin proteolysate or the peptide (VVYP) is not limited insofar as it is sufficient for exhibiting an action of inhibiting an increase in blood sugar or an action of enhancing insulin secretion. Usually, the pharmaceutical composition is prepared concomitantly with pharmacologically acceptable carriers and additives.

Examples of carriers are excipients, diluents, binders, moisturizers, disintegrators, disintegration inhibitors, absorption enhancers, lubricants, auxiliary dissolvents, buffers, emulsifiers, suspending agents, and the like that are typically used according to the form of administering the pharmaceutical composition (preparation). Examples of additives are stabilizers, preservatives, buffers, isotonicity adjusters, chelating agents, pH-adjusters, surfactants, coloring agents, aroma chemicals, flavoring agents, sweeteners, and the like that are typically used according to the form of administering the preparation.

The unit dosage form of the pharmaceutical composition (form of pharmaceutical preparation) can be suitably selected according to the administration route. The pharmaceutical composition may be roughly classified as an oral agent, transpulmonary agent, transnasal agent, sublingual agent, parenteral agent (injection, drip), or the like. The pharmaceutical composition can be compounded, formed or prepared according to known methods into solid dosage forms such as tablets, pills, dispersants, powders, granules, capsules, etc.; and liquid dosage forms such as solutions, suspensions, emulsions, syrups, elixirs, etc. The pharmaceutical composition may be prepared in the form of a dried product that can be liquefied by the addition of a suitable carrier when used. The pharmaceutical composition can be prepared into any such forms according to known methods.

The proportion of the globin proteolysate or the peptide (VVYP) in the pharmaceutical composition is not limited. Usually, the pharmaceutical composition is formed into a preparation form that contains the globin proteolysate in about 0.1 to about 80 wt.%, preferably about 5 wt.% to about 60 wt.%, and more preferably about 20 wt% to about 50 wt% wt% or the peptide (VVYP) in about 0.001 to about 80 wt.%, preferably about 0.01 wt.% to about 10 wt%.

The dosage of the pharmaceutical composition obtained in such a manner can be suitably determined according to the purpose of the pharmaceutical composition (for inhibiting an increase in blood sugar, enhancing insulin secretion, preventing or treating diseases resulting from hyperglycemia, etc.); method of administering the pharmaceutical composition; mode of administration; age, body weight, symptoms (severity of diabetes) of the patient; and other factors. It is usually preferable that the globin proteolysate is administered to an adult in a daily dosage of about 100 to about 2000 mg, or the peptide (VVYP) is administered to an adult in a daily dosage of about 1 to about 20 mg.

The pharmaceutical composition does not have to be administered in a single dose, and can be administered in 3 to 4 divided doses per day. The pharmaceutical preparation in such an aforementioned form is administered in a route suitable for the form, for example, the pharmaceutical preparation in an injectable form can be administered intravenously, intramuscularly, subcutaneously, intracutaneously, intraperitoneally, or like manner; the pharmaceutical preparation in a solid form can be administered orally or like manner.

As demonstrated in the Examples below, the pharmaceutical composition has an action of enhancing insulin secretion due to the globin proteolysate or the peptide (VVYP), ameliorates a hyperglycemic condition caused by the weakening or lack of the action of insulin, and hence exhibits a hypoglycemic action. Therefore, the pharmaceutical composition is of use as a composition for preventing or treating various diseases resulting from hyperglycemic conditions caused by the weakening or lack of the action of insulin.

Diabetes and diabetic complications are included within such diseases. The target diabetes is preferably non-insulin-dependent type II diabetes. Diabetic complications discussed herein refer to systemic and local diseases developed directly or indirectly with diabetes (preferably non-insulin-dependent type II diabetes). Specific examples are diabetic acidosis, diabetic xanthoma, diabetic amyotrophy, diabetic ketosis, diabetic coma, diabetic gastric disorder, diabetic gangrene, diabetic ulcer, diabetic diarrhea, diabetic microangiopathy, diabetic uterine body sclerosis, diabetic cardiomyopathy, diabetic neuropathy, diabetic nephropathy, diabetic bulla, diabetic cataract, diabetic dermopathy, diabetic scleredema, diabetic retinopathy, necrobiosis lipoidica diabeticorum, diabetic blood circulation disorder, etc.

### (2) Food composition

Food compositions provided according to the present invention include foods for specified health use (including foods qualified for specified health use) that have a function of inhibiting an increase of blood sugar, and are preferably used with an eye to such a function; foods for specified health use (including foods qualified for specified health use) that have a function of enhancing insulin secretion, and are preferably used with an eye to such a function; and foods for specified health use (including foods qualified for specified health use) for use in preventing or treating a disease resulting from hyperglycemia due to a blood sugar increase inhibitory action or insulin secretion enhancing action.

A feature of the aforementioned foods for specified health use having a function of inhibiting an increase in blood sugar is containing the globin proteolysate or the peptide (WYP) in an amount effective for preventing or ameliorating hyperglycemia (inhibiting an increase in blood sugar) in a diabetic patient or borderline diabetic patient. Packaging and advertisements for the foods may carry a notice of the effects (action of inhibiting an increase in blood sugar). A feature of the foods for specified health use having a function of enhancing insulin secretion is containing the globin proteolysate or the peptide (WYP) in an amount effective for enhancing insulin secretion in a diabetic patient or borderline diabetic patient. Packaging and advertisements for the foods may carry a notice of the effects (enhancement in insulin secretion). A feature of the foods for specified health use for use in preventing or treating a disease resulting from hyperglycemia is containing the globin proteolysate or the peptide (WYP) in an amount effective for exhibiting an action of inhibiting an increase in blood sugar or action of enhancing insulin secretion. Packaging and advertisements for the foods may carry a notice of the effects (antidiabetic action).

The food composition of the present invention may be composed entirely of the globin proteolysate or the peptide (VVYP). When the food composition is prepared in combination with other substances, the amount of the globin proteolysate or the peptide (VVYP) is not limited insofar as it is sufficient for exhibiting an action of inhibiting an increase in blood sugar or an action of enhancing insulin secretion. The food composition of the present invention may be prepared concomitantly with carriers and additives that are usable in foods. Such food compositions include supplements and the like prepared by mixing the globin proteolysate or the peptide (WYP) with carriers and additives that are acceptable in foods, in the form of tablets, pills, capsules, granules, dispersions, powders, solutions (drinks), etc. Also, the food composition of the present invention includes products containing the globin proteolysate or the peptide (VVYP) that may take the form of ordinary foods and drinks.

Examples of such foods and drinks include milk beverages, lactic acid bacteria beverages, fruit juice-containing soft drinks, carbonated beverages, fruit juice drinks, vegetable juice drinks, vegetable/fruit beverages, alcoholic beverages, powdered beverages, coffee beverages, black tea beverages, green tea beverages, barley tea beverages, and like beverages; custard puddings, milk puddings, soufflé puddings, fruit juice-containing puddings and like puddings, jellies, bavarois, yogurt, and like desserts; ice creams, ice milks, lacto-ices, milk ice creams, fruit juice-containing ice creams, soft creams, ice candies, sherbets (sorbets), frozen confections, and like chilled confections; chewing gums, bubble gums, and like gums (stick gums, sugar-coated tablet gums); marble chocolates and like coated chocolates, strawberry chocolates, blueberry chocolates, melon chocolates and like flavored chocolates, and like chocolates; hard candies (including bonbons, butterballs, marbles, etc.), soft candies (including caramels, nougats, gummy candies, marshmallows, etc.), drops, taffy, and like caramels; hard biscuits, cookies, *okaki* (rice crackers), *sembei* (rice crackers), and like baked confections (all of the above are confections); consommé soups, potage soups, and like soups; strawberry jams, blueberry jams, marmalades, apple jams, apricot jams, preserves, like jams; red wines and like fruit wines; syruped cherries, apricots, apples, strawberries and peaches, and like processed fruits; ham, sausage, roast pork, and like processed livestock meat; fish ham, fish sausage, fish fillets, *kamaboko* (steamed fish paste), *chikuwa* (baked fish paste), *hanpen* (minced and steamed fish), *satsumaage* (fried fish ball), *datemaki* (omelet wrappers), whale bacon, and like processed marine products; *udon* (wheat noodle), *hiyamugi, somen* (fine noodle), *soba* (buckwheat noodle), Chinese noodle, spaghetti, macaroni, *bifun* (rice noodle), *harusame* (bean-jelly stick), wonton, and like noodles; and various types of side dishes, wheat gluten cake, *denbu,* and like various other processed food products. The food composition of the invention is preferably in the form of a beverage or a confection.

The amount of active ingredient (the globin proteolysate or the peptide (VVYP)) in the food composition, or the dosage of the food composition, is not limited, and can be suitably selected from a broad range according to the type of the food composition, extent of the desired ameliorative effect, and other factors. The dosage of the food composition, although varying depending on the type of the food composition, can be suitably selected from the range of about 100 to about 2000 mg/60 kg calculated as globin proteolysate, or from the range of about 1 to about 20 mg/60 kg calculated as peptide (VVYP), per administration to a human of 60 kg body weight.

The food composition of the present invention has an action of enhancing insulin secretion due to the globin proteolysate or the peptide (VVYP), ameliorates hyperglycemic conditions brought about by the weakening or lack of the action of insulin, and hence exhibits a hypoglycemic action. Therefore, the food composition of the present invention is of use as a composition for preventing or treating various diseases resulting from hyperglycemic conditions brought about by the weakening or lack of the action of insulin.

### (3) Feed composition

The feed composition provided according to the present invention includes a feed that has a function of inhibiting blood sugar increase; a feed that has a function of enhancing insulin secretion; and a feed for use in preventing or treating a disease resulting from hyperglycemia due to the action of inhibiting an increase in blood sugar or enhancing insulin secretion. Such feeds can be preferably used as pet food especially for dogs, cats and like animals. A feature of the aforementioned feed with the function of inhibiting an increase in blood sugar is containing the globin proteolysate or the peptide (WYP) in an amount effective for preventing or ameliorating hyperglycemia (inhibiting a blood sugar increase) in a diabetic animal or borderline diabetic animal. A feature of the feed with the function of enhancing insulin secretion is containing the globin proteolysate or the peptide (VVYP) in an amount effective for enhancing insulin secretion in a diabetic animal or borderline diabetic animal. A feature of the feed for use in preventing or treating a disease resulting from hyperglycemia is containing the globin proteolysate or the peptide (WYP) in an amount effective for exhibiting an action of inhibiting an increase in blood sugar or enhancing insulin secretion.

Such feed compositions include those prepared by mixing the globin proteolysate or the peptide (VVYP) with carriers and additives that can be used in feeds, in the form of tablets, pills, capsules, granules, dispersions, powders, solutions, etc. Also, the feed composition of the present invention includes products containing the globin proteolysate or the peptide (WYP) that may take the form of ordinary feeds.

The amount of active ingredient (the globin proteolysate or the peptide (VVYP)) contained in the feed composition, or the dosage of the feed composition, is not limited, and can be suitably selected from a broad range according to the type of the feed composition, kind of animal that takes the feed composition, extent of the desired ameliorative effect, and other factors. The dosage of the feed composition, although varying depending on the type of the feed composition, can suitably be selected from the range of about 50 to about 1000 mg/10 kg calculated as globin proteolysate, or from the range of about 0.5 to about 10 mg/10 kg calculated as peptide (VVYP), per administration to an animal of 10 kg body weight.

### Examples

Preparation Examples and Test Examples are given below to illustrate the invention in more detail, but the scope of the invention is not limited by these examples. In the following Test Examples, "%" represents "percent by weight", unless specified otherwise.

### Preparation Example 1 Preparation of globin proteolysate

A method for preparing a globin proteolysate using bovine erythrocyte is described below in detail.

To 100 kg of fresh bovine erythrocytes was added 250 liters of water to allow sufficient hemolysis. After adjustment of the pH to 2.8 with phosphoric acid, 2.6 x 10⁷ units of acid protease from *Aspergillus niger* were added to the solution and reacted at 50°C for 3 hours.

After the reaction, the reaction solution was heated at 80°C for 30 minutes to terminate the reaction. Thereafter, an aqueous suspension of calcium hydroxide was added to the reaction solution to adjust the pH to 6.5. Then, 10 kg of diatomaceous earth was added, and the mixture was filtered with a filter press. The resulting filtrate was spray-dried, thereby producing 23 kg of a globin proteolysate as a powder. The molecular weight distribution of the globin proteolysate was examined by gel filtration chromatography performed under the following conditions.

### <Gel filtration chromatography>

Equipment: High-performance liquid chromatograph (Shimadzu Corporation, Model LC-6A)
Column: PolyHYDROXYETHYL A, 5 µm, 9.4 x 200 mm, manufactured by PolyLC Inc.
Eluate: 50 mM Formic acid
Flow rate: 0.5 ml/min
Detection: UV absorption at 221 nm

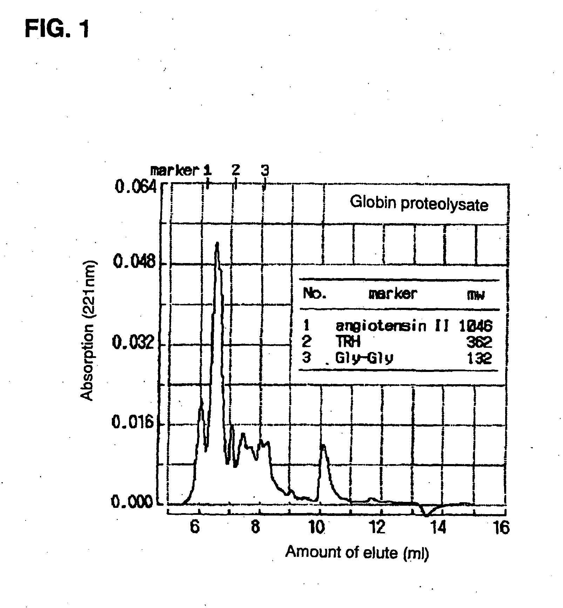

Figure 1 shows a gel filtration chromatogram of a globin proteolysate obtained according to the above-described gel filtration chromatography.

### Preparation Example 2 Fractionation and purification of peptide that inhibits an increase in blood TG levels

The peptide of the present invention was obtained through the following procedures: (1) ion exchange, (2) ultrafiltration, (3) separation by reverse phase column chromatography under acidic conditions, and (4) separation by reverse phase chromatography under neutral conditions.

### (1) Ion exchange

A 10% aqueous solution containing 13.7 g of the globin proteolysate obtained in Preparation Example 1 was introduced to a weakly acidic cation exchange resin (Amberlite IRC₅₀, H⁺ Type, Organo Co., Ltd.) and stirred for 1 hour for adsorption. An unadsorbed fraction was thereby separated.

### (2) Ultrafiltration

The unadsorbed fraction obtained by the aforementioned ion exchange was subjected to ultrafiltration using a stirring-force ultrafilter unit (manufactured by Advantec, UHP 90K) and a ultrafilter membrane (manufactured by Advantec, UIIH-1, molecular weight cutoff: 1000). The liquid remaining on the ultrafilter membrane (residual liquid) was collected. The fraction thus obtained was subjected to acid hydrolysis and quantified according to a ninhydrin method. The acid hydrolysis was carried out by placing 1 ml of hydrochloric acid having a final concentration of 6 N into a test tube per 3 to 5 mg of protein, sealing the tube under atmospheric pressure and heating it at 110°C for 22 hours. The aforementioned ninhydrin method was performed as follows. The pH of the specimen after hydrolysis was adjusted to 5.0 with sodium hydroxide. The specimen was then reacted with a ninhydrin reagent dissolved in a 0.2 M citrate buffer (pH 5.0) at 100°C for 15 minutes. Absorbance at 570 nm was measured. Separately, aqueous L-leucine solutions (75, 150, 225, and 300 nmol/ml) were subjected to a ninhydrin reaction as standard solutions. A calibration curve was obtained from the absorbances measured, and the amount of amino groups equivalent to L-leucine in the specimen was calculated. The result of the quantification is presented in Table 1. The yield based on the starting globin proteolysate is also presented in Table 1.

### (3) Reverse phase (acid) chromatography

The filtrate obtained after the ultrafiltration above was subjected to reverse phase (acid) chromatography under the following conditions.

### <Reverse phase (acid) chromatography>

Equipment: High performance liquid chromatograph (Shimadzu Corporation, Model LC-10A)
Column: SuperPac Pep-S, 15 µm. 22.5 x 250 mm, manufactured by Pharmacia)
Eluate: Aqueous acetonitrile solution containing 0.1% trifluoroacetic acid.
   Linear concentration gradient of acetonitrile from 2 to 35%; Acetonitrile concentration was changed at a rate of 1%/min
Flow rate: 5 ml/min
Temperature: 40°C
Detection: UV absorption at 220 nm
Fractioning Time: 53.8 to 54.5 minutes (Fraction A)

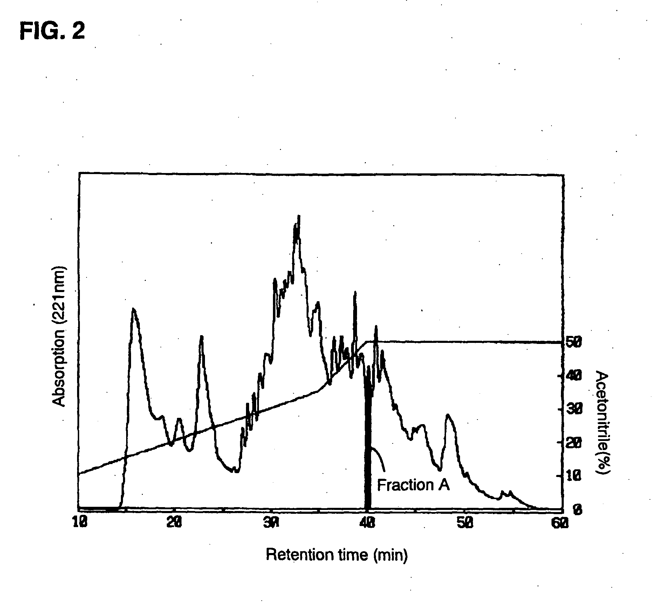

Figure 2 shows a chromatogram obtained by the above-described reverse phase (acid) chromatography.

The fraction thus obtained was subjected to acid hydrolysis and then quantified by amino acid analysis. Acid hydrolysis was carried out by placing 1 ml of hydrochloric acid (final concentration of 6 N HCl) into a test tube per 3 to 5 mg of protein, sealing the test tube under reduced pressure, and heating it at 110°C for 22 hours. Amino acid analysis was conducted under the following conditions.

### <Amino acid analysis>

Equipment: High performance liquid chromatograph (Shimadzu Corporation, Model LC-6A)
Column: Shim-pack ISC-07/S1504 Na, 7 µm, 4.0 x 150 mm, manufactured by Shimadzu Corporation
Eluate: Amino acid mobile phase kit (Na type) manufactured by Shimadzu Corporation
Flow rate: 0.3 ml/min
Temperature: 55°C

### Reaction solution 1: Analysis kit OPA reagent manufactured by Shimadzu Corporation

### Detection: Fluorescence absorption (Ex 348 nm. Em 450 nm)

The acid-hydrolyzed solution was concentrated, dried and caked using a rotary evaporator, and further dried under reduced pressure for more than 12 hours, thereby completely removing hydrochloric acid. The resultant was dissolved in 0.2 M citrate buffer (pH 2.2) such that each amino acid was contained in a proportion of about 100 nmol/ml. This solution was filtered through a 0.45 µm filter, and 10 µl of the filtrate was introduced into the column. For a standard solution, an 18-component type-H amino acid mixed standard solution (Wako Pure Chemical Industries, Ltd.) was diluted 25-fold with 0.2 M citrate buffer (pH 2.2), and 10 µl of the diluted solution was introduced into the column (1 nmol/10 µl for each amino acid). The peak areas of the amino acids were calculated and analyzed using Chromatopac C-R4A (Shimadzu Corporation), and the amounts of amino acids were calculated based on the ratios of the peak areas for the specimen to the peak areas for the standard solution. The results are shown in Table 1. The yield based on the globin proteolysate is also presented in Table 1.

### (4) Reverse phase (neutral) chromatography

The fraction eluted and fractioned by the above-described reverse phase (acid) chromatography was further subjected to reverse phase (neutral) chromatography under the following conditions.

### <Reverse phase (neutral) chromatography>

Equipment: High performance liquid chromatograph (Shimadzu Corporation, Model LC-10A)
Column: SuperPac Pep-S, 15 µm, 22.5 x 250 mm, manufactured by Pharmacia)
Eluate: Aqueous acetonitrile solution containing 20 mM ammonium acetate buffer (pH 6.5)
   Linear concentration gradient of acetonitrile from 0 to 25%; Acetonitrile concentration was changed at a rate of 0.5%/min
Flow rate: 5 ml/min
Temperature: 40°C
Detection: UV absorption at 220nm
Fractioning Times: 41.7 to 43.2 minutes (Fraction B), 45.8 to 51.0 minutes (Fraction C).

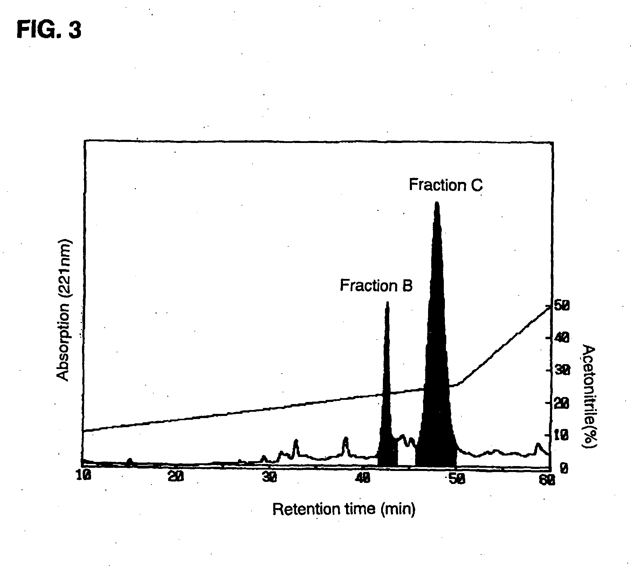

Figure 3 shows a chromatogram obtained by the reverse phase (neutral) chromatography described above. The resulting fractions were quantified as in (3) above, and then identified. The amino acid composition was computed based on the proportion of each amino acid relative to the total amino acid content. The results showed that Fraction B was VTL (Val-Thr-Leu), and Fraction C was VVYP (Val-Val-Tyr-Pro). Matching with the amino acid sequence of hemoglobin verified that these fractions are present in hemoglobin. The results of the quantification are presented in Table 1 together with the yields based on the globin proteolysate.

**Table 1**

| Peptide | Amount of protein (g) | Yield (%) |
|---|---|---|
| Globin proteolysate | 13.70 | 100.0 |
| Ion exchange + Ultrafiltration | 4.24 | 30.9 |
| Reverse phase chromatography | | |
| Fraction A | 0.39 | 0.28 |
| Fraction B VTL | 0.009 | 0.06 |
| Fraction C WYP | 0.006 | 0.04 |

### Test Example 1 Inhibitory effect on an increase in blood sugar level (mice)

A sugar tolerance test was carried out using the globin proteolysate obtained in Preparation Example 1 and the Fraction C peptide (WYP) obtained in Preparation Example 2(4) to investigate their inhibitory effects on an increase in blood sugar levels.

Seventy male ICR mice (8 weeks old) that had been fasted overnight were divided into 7 groups consisting of 10 mice per group. Using an oral probe, the mice of each group were intragastrically administered with the globin proteolysate in an amount of 5 mg (Test Group 1), 10 mg (Test Group 2) or 50 mg (Test Group 3); the peptide (WYP) in an amount of 1 µg (Test Group 4), 2 µg (Test Group 5) or 10 µg (Test Group 6); or water in an amount of 0.2 ml (Control Group). The globin proteolysate and the peptide (WYP) were used after being dissolved in 0.2 ml of water. Immediately after the administration, 0.2 ml of a glucose solution (0.25 g/ml aqueous glucose solution) was intragastrically administered in a similar manner using an oral probe. Blood was collected from the tail veins 30, 60, 90 and 120 minutes after the administration of the glucose solution, and the blood sugar levels were measured using a reagent kit (Glucose CII Test Wako, Wako Pure Chemical Industries, Ltd., Osaka) according to a mutarotase/GOD method.

The blood sugar levels (AUC: area under the curve, mg/dL·120min) of the test groups and the control group 120 minutes after the administration of the glucose solution are presented in Table 2. In Table 2, "percentage relative to control" refers to the proportion of the blood sugar level of a test group relative to the blood sugar level of the control group being 100% (the same applies hereinbelow),

**Table 2**

| Group | Dosage (amount/mouse) | | Blood sugar level (mg/dL·120min) | Percentage relative to control |
|---|---|---|---|---|
| Control Group | - | | 1768 ± 360 | 100% |
| Test Group 1 | Globin proteolysate | 5 mg | 1654 ± 470 | 94% |
| Test Group 2 | | 10 mg | 1596 ± 500 | 90% |
| Test Group 3 | | 50 mg | 888 ± 204* | 50% |
| Test Group 4 | Peptide (WYP) | 1 µg | 1537 ± 486 | 87% |
| Test Group 5 | | 2 µg | 892 ± 210* | 54% |
| Test Group 6 | | 10 µg | 644 ± 140** | 40% |

| | | | | |
|---|---|---|---|---|
| * p<0.05 ** p<0.01 | | | | |

As can be understood from the table, the globin proteolysate and the peptide (WYP) significantly inhibited an increase in blood sugar levels in the mice in a dose dependent manner. The activity of reducing blood sugar of the peptide (WYP) is more than 1000 times stronger than that of the globin proteolysate. Since the globin proteolysate contains the peptide (WYP) in a proportion of about 1%, one of the active ingredients (active components) of the globin proteolysate that gives the action of reducing blood sugar is presumably the peptide (WYP).

### Test Example 2 Inhibitory effect on an increase in blood sugar level (Japanese)

A sugar tolerance test (7-day drug holidays, single-blind crossover trial) was carried out using the globin proteolysate obtained in Preparation Example 1 for humans (Japanese) (diabetic subjects: 12 type II diabetes patients who had fasting blood sugar levels of 200 mg/dl or greater; normal subjects: 10 normal subjects who had fasting blood sugar levels of 100 mg/dl or less; average body weight: 68 kg) to investigate the action of inhibiting an increase in blood sugar levels of the globin proteolysate.

The subjects (diabetic subjects and normal subjects) in a hunger state due to fasting since the previous night were given the globin proteolysate in an amount of 1000 mg, 1500 mg or 3000 mg (test groups), or milk casein in an amount of 3000 mg (control groups). Immediately after the administration, a glucose solution (75 g/200 ml) was administered. Blood was collected 60, 90 and 120 minutes after the administration of the glucose solution, and the blood sugar levels were measured using a reagent kit (Glucose CII Test Wako, Wako Pure Chemical Industries, Ltd., Osaka) according to a mutarotase/GOD method.

The blood sugar levels (AUC: area under the curve, mg/dL·120min) of the subjects of the test groups and the control groups (diabetic subjects and normal subjects) 120 minutes after the administration of a glucose solution are presented in Table 3.

**Table 3**

| Subject | Dosage | | Blood sugar level (mg/dL·120min) | Percentage relative to control |
|---|---|---|---|---|
| Normal subjects (n = 10) | Control Group | 0 mg | 4575 ± 680 | 100% |
| | Test Groups | | | |
| | Globin proteolysate | 1000 mg | 4212 ± 754 | 92% |
| | Globin proteolysate | 1500 mg | 4198 ± 770 | 92% |
| | Globin proteolysate | 3000 mg | 3745 ± 759** | 82% |
| Diabetic subjects (n = 12) | Control Group | 0 mg | 12675 ± 1860 | 100% |
| | Test Groups | | | |
| | Globin proteolysate | 1000 mg | 11431 ± 2288 | 90% |
| | Globin proteolysate | 1500 mg | 10862 ± 2203* | 86% |
| | Globin proteolysate | 3000 mg | 8773 ± 2480** | 69% |

| | | | | |
|---|---|---|---|---|
| * p<0.05 ** p<0.01 | | | | |

As can be understood from the table, the administration of the globin proteolysate in an amount of 3000 mg showed statistically significant inhibitory effect on an increase in blood sugar levels for both the type II diabetic subjects and the normal subjects. However, in light of clinical application, globin proteolysate will exhibit more significant, useful effects on diabetic subjects than normal subjects.

Hence, the globin proteolysate can be considered as significantly inhibiting an increase in blood sugar in subjects with type II (non-insulin-dependent) diabetes. In view of the results of Test Example 1 above, the principal component (active ingredient) of the globin proteolysate is believed to be the peptide (VVYP), and the peptide (WYP) is expected to inhibit an increase in blood sugar in patients with type II (non-insulin-dependent) diabetes with an activity about 5000 times stronger than that of the globin proteolysate.

### Test Example 3 Increasing effect on blood insulin level (Japanese)

Twelve subjects (Japanese, average body weight: 67 kg) were administered with the globin proteolysate obtained in Preparation Example 1 in amounts of 1000 and 3000 mg and a placebo (milk casein) in an amount of 1000 mg with 7-day drug holidays (single-blind crossover trial). After the administration of each substance, blood was collected to measure the blood insulin and blood triiodothyronine levels. In particular, the subjects were fasted for more than 12 hours from the previous night. On the test day, blood was collected every hour from immediately before the administration to 6 hours after the administration to measure the blood insulin and blood triiodothyronine levels. The blood insulin levels were measured using a "Glazyme Insulin-EIA TEST" (Wako Pure Chemical Industries, Ltd., Osaka). The measurement of the blood triiodothyronine levels (according to the CLIA method) was assigned to Shionogi Biomedical Laboratories (Osaka).

The blood insulin levels (mU/L·3h) and the blood triiodothyronine levels (ng/L·3h) 3 hours after the administration of the test substances (the globin proteolysate in 1000 mg and 3000 mg, and placebo) are presented in Table 4.

**Table 4**

| | Placebo intake | Globin proteolysate intake | |
|---|---|---|---|
| | | 1000 mg | 3000 mg |
| Blood insulin level (mU/L·3h) | 8.7 ± 1.1 | 10.1 ± 1.6 | 15.6 ± 2.1* |
| | 100% | 116% | 179% |
| Blood triiodothyronine level (ng/L·3h) | 0.38 ± 0.10 | 0.22 ± 0.08 | 0.33 ± 0.07 |

| | | | |
|---|---|---|---|
| * p<0.05 | | | |

As can be understood from the table, the administration of the globin proteolysate in an amount of 3000 mg did not make much difference in blood triiodothyronine levels but blood insulin levels (rate of insulin secretion) were significantly increased. This fact establishes that the globin proteolysate has an action of specifically enhancing the rate of insulin secretion in humans (Japanese) without adversely affecting the function of the thyroid gland. Moreover, considering the results presented in the table, the effect for inhibiting an increase in blood sugar of the globin proteolysate in humans (Japanese) shown in Test Example 2 is presumably due to its action of enhancing insulin secretion, and considering the results of Test Example 1, the active component (active ingredient) of the globin proteolysate is presumably the peptide (VVYP).

### Test Example 4 Increasing effect on blood insulin level (Westerners)

Seventeen westerners (7 obese subjects, 10 normal subjects, average body weight: 82 kg) were administered with the globin proteolysate prepared in Preparation Example 1 in amounts of 500, 1000 and 2000 mg and a placebo (milk casein in an amount of 1000 mg) with 7-day drug holidays (single-blind crossover trial). After the administration of each substance, blood was collected to measure the blood insulin and blood sugar levels. In particular, the subjects were given a cream soup (containing 41 g of carbohydrate and 67 g of fat) concurrently with a test substance. Blood was collected every hour from immediately before (0) to 4 hours after the administration to measure the blood insulin and blood sugar levels.

The blood insulin levels were measured using a "Glazyme Insulin-EIA TEST" (Wako Pure Chemical Industries, Ltd., Osaka). The blood sugar levels were measured using a reagent kit (Glucose CII Test Wako, Wako Pure Chemical Industries, Ltd., Osaka) according to a mutarotase/GOD method.

The blood insulin levels (AUC: area under the curve, ?mU/L·4h) and the blood sugar levels (?mg/dL·4h) 4 hours after the administration of the test substances (the globin proteolysate in 500 mg, 1000 mg and 2000 mg, and the placebo) to the obese subjects and normal subjects are presented in Table 5.

**Table 5**

| Effect of globin proteolysate on blood insulin levels | | | | | | | | |
|---|---|---|---|---|---|---|---|---|
| Subject | Test substance | Dosage | Insulin | | | Blood sugar level | | |
| | | (mg) | (?mU/L·4h) | | | (?mg/dL·4h) | | |
| Obese subject | Placebo | 0 | 47.9 ± 7.1 | 100% | | 42.2 ± 9.6 | 100% | |
| | Globin proteolysate | 500 | 73.4 ± 10.5 | 153% | p<0.05 | 40.9 ± 6.7 | 97% | |
| | | 1000 | 85.2 ± 12.8 | 178% | p<0.01 | 24.2 ± 4.9 | 57% | p<0.05 |
| | | 2000 | 64.9 ± 4.4 | 136% | p<0.05 | 30.0 ± 5.2 | 71% | p<0.05 |
| Normal subject | Placebo | 0 | 51.2 ± 3.6 | 100% | | 38.7 ± 11.3 | 100% | |
| | Globin proteolysate | 500 | 55.7±5.4 | 109% | | 34.1 ± 8.2 | 88% | |
| | | 1000 | 40.2 ± 2.7 | 79% | p<0.01 | 39.6 ± 7.3 | 102% | |
| | | 2000 | 42.4 ± 2.7 | 83% | p<0.01 | 38.8 ± 6.4 | 100% | |

As can be understood from the table, the blood insulin levels (rates of insulin secretion) of the obese subjects were clearly increased by the administration of the globin proteolysate, and the increase in blood sugar level was inhibited accordingly. This effect was significant when the globin proteolysate was administered in an amount of 1000 mg. In contrast, no increase (enhancement) in blood insulin levels (rate of insulin secretion) was observed in the normal subjects. The normal subjects rather showed a downward trend.

In Test Example 3 carried out on Japanese subjects, the administration of the globin proteolysate in an amount of 3000 mg showed a greater effect for enhancing blood insulin levels (rates of insulin secretion) than the administration of the globin proteolysate in an amount of 1000 mg. In contrast, in this Test Example carried out on Westerners, the administration of the globin proteolysate in an amount of 1000 mg showed a greater effect for enhancing blood insulin levels (effect for enhancing insulin secretion) than the administration of the globin proteolysate in an amount of 2000 mg. Considering this fact, while the globin proteolysate is usable as an effective ameliorative or preventive composition for diabetic Japanese, it is presumably more effective for diabetic Westerners.

### Test Example 5 Lowering effect on blood sugar level (Westerners)

Thirty obese subjects having a BMI of 30 or greater (ages 18 to 65) were subjected to a 12-week obesity treatment program (alimentary therapy, exercise therapy). Seventeen randomly selected subjects among them consumed once a day at home a low-calorie food containing 1.5 g of the globin proteolysate prepared in Preparation Example 1 (test group). The remaining 13 subjects consumed once a day at home a low-calorie food that did not contain the globin proteolysate (control group). After 37 weeks of this home healthcare, the fasting blood sugar levels and body weights of the test subjects of the test group and the control group were measured to make a comparison with those measured before the beginning of the home healthcare. The blood sugar levels were measured using a reagent kit (Glucose CII Test Wako, Wako Pure Chemical Industries, Ltd., Osaka) according to a mutarotase/GOD method. Table 6 shows the averages of change in the fasting blood sugar levels relative to the fasting blood sugar levels before the beginning of the home healthcare of the test subjects of the test group and the control group.

**Table 6**

| | Number of subjects | Change in body weight (kg) | Change in blood sugar level (mg/dL) |
|---|---|---|---|
| Control group | 13 | -3.7 ± 7.2 | +9.0 ± 14.1 |
| Test group (globin proteolysate-administered group) | 17 | -3.9 ± 9.4 | -7.3 ± 16.8 |

As can be understood from the table, the test subjects from both the globin proteolysate-administered group and non-globin-proteolysate-administered group (control group) showed body weights lower than those before the beginning of the home healthcare. Moreover, the globin proteolysate-administered group showed significantly lowered blood sugar levels. In view of the results of Test Example 4, the action of reducing blood sugar of the globin proteolysate shown in this test example is presumably due to enhanced insulin secretion.

### Test Example 6 Safety Test

The peptide having the amino acid sequence Val-Val-Tyr-Pro (SEQ. ID. No. 1) prepared in Example 2 was orally administered to male and female ICR mice in an amount of 10 g/kg (maximum allowable dose) or greater, and there were no fatalities, thereby establishing the safety of the peptide.

### Example 1 Preparation of peptide (VVYP)-containing food (1) Preparation of powdered milk

Ten milligrams of the peptide having the amino acid sequence Val-Val-Tyr-Pro (SEQ. ID. No. 1) prepared in Preparation Example 2 was added to 100 g of infant powdered milk formula, thereby giving a powdered milk having a function of enhancing insulin secretion or inhibiting an increase in blood sugar.

### (2) Preparation of chocolate

Fifty milligrams of the peptide having the amino acid sequence Val-Val-Tyr-Pro (SEQ. ID. No. 1) prepared in Preparation Example 2 was added to 100 g of chocolate, thereby giving a chocolate having a function of enhancing insulin secretion or of inhibiting an increase in blood sugar.

### (3) Preparation of green tea beverage

Eight kilograms of green tea leaves were introduced to 300 1 of hot water (80°C) and extracted at that temperature for 4 minutes. The resulting extract was cooled and centrifuged. Clear supernatant was collected as a green tea extract. To this extract were added 0.4 kg of vitamin C and then 50 g of the peptide having the amino acid sequence Val-Val-Tyr-Pro (SEQ. ID. No. 1) prepared in Preparation Example 2. Hot water was added to attain the final volume of 1000 1. The mixture was heated to 85° C or higher, charged into a metal can, and retort-sterilized (125°C, 5 minutes), thereby giving a green tea beverage.

### (4) Preparation of chewing gum

A chewing gum was prepared using the formulation below.

**<Chewing gum formulation>**

| | |
|---|---|
| Gum base | 25.0 parts by weight |
| Sucrose | 63.8 parts by weight |
| Corn syrup | 10.0 parts by weight |
| Glycerol | 1.0 part by weight |
| Peptide (WYP) | 0.2 parts by weight |
| Total | 100.0 parts by weight |

Gum base, sugar, corn syrup and glycerol were mixed first. The peptide having the amino acid sequence Val-Val-Tyr-Pro (SEQ. ID. No. 1) prepared in Preparation Example 2 was then added, and the mixture was uniformly kneaded using a mixer at 50°C. After cooling, the mixture was press-molded by a roller, thereby giving a chewing gum stick containing, as an active ingredient, the peptide (VVYP) in a proportion of 5 mg per stick.

### Example 2 Preparation of globin proteolysate-containing food (1) Supplement

The following ingredients were kneaded, granulated, dried, and tableted according to a standard method, thereby producing tablets containing, as an active ingredient, globin proteolysate in an amount of 25 wt.% (50 mg) per tablet (200 mg). These tablets are for use as supplements having pharmacological actions resulting from the globin proteolysate (antidiabetic action, action of inhibiting an increase in blood sugar levels, action of enhancing insulin secretion).

| < Tablet formulation > | (per tablet) |
|---|---|
| Globin proteolysate (Preparation Example 1) | 50 mg |
| Crystalline cellulose | 140 mg |
| Sucrose fatty acid ester | 10 mg |
| Total | 200 mg |

### Example 3 Preparation of feed containing the peptide of the present invention

The peptide having the amino acid sequence Val-Val-Tyr-Pro (SEQ. ID. No. 1) prepared in Preparation Example 2 was added in a proportion of 0.1 wt.% to a premix containing vitamins, minerals and the like. This mixture was added to a commercially available dog food in a proportion of 10 wt.%, thereby producing a dog food having a function of enhancing insulin secretion or inhibiting an increase in blood sugar.

According to the present invention, an antidiabetic agent, food composition and feed composition can be produced which exhibit an action of reducing blood sugar or enhancing insulin secretion due to a globin proteolysate or a peptide (WYP) contained therein as an active ingredient. Such compositions exhibit an action of lowering hyperglycemic blood sugar levels in diabetic and borderline diabetic subjects due to the action of reducing blood sugar or enhancing insulin secretion. Therefore, the compositions of the present invention can be effectively used in preventing or treating diseases resulting from hyperglycemia, in particular, diabetes and diabetic complications.

## Claims

1. Use of a peptide having the sequence Val-Val-Tyr-Pro (SEQ-ID NO:1) or a globin proteolysate containing the peptide for preparing a food composition for preventing or treating disease resulting from hyperglycemia.

2. Use of a peptide having the sequence Val-Val-Tyr-Pro (SEQ-ID NO:1) or a globin proteolysate containing the peptide for preparing a feed composition for preventing or treating disease resulting from hyperglycemia.

3. Use of a peptide having the sequence Val-Val-Tyr-Pro (SEQ-ID NO:1) or a globin proteolysate containing the peptide for preparing an antidiabetic agent.

4. Use of a peptide having the sequence Val-Val-Tyr-Pro (SEQ-ID NO:1) or a globin proteolysate containing the peptide for preparing a food for specified health use that has a function of inhibiting an increase in blood sugar or a function of enhancing insulin secretion and is used with an eye to such a function.

5. Use of a peptide having the sequence Val-Val-Tyr-Pro (SEQ-ID NO:1) or a globin proteolysate containing the peptide for preparing a feed for specified health use that has a function of inhibiting an increase in blood sugar or a function of enhancing insulin secretion and is used with an eye to such a function.

6. A peptide having the sequence Val-Val-Tyr-Pro (SEQ-ID NO:1) or a globin proteolysate containing the peptide for use in the prevention or treatment of a disease resulting from hyperglycemic condition caused by the weakening or lack of the action of insulin.

7. A peptide having the sequence Val-Val-Tyr-Pro (SEQ-ID NO:1) or a globin proteolysate containing the peptide for use in the prevention or treatment of a disease resulting from hyperglycemic condition caused by the weakening or lack of the action of insulin according to claim 6, wherein the disease is diabetes or a diabetic complication.

## Patentansprüche

1. Verwendung eines Peptids, das die Sequenz Val-Val-Tyr-Pro (SEQ-ID Nr.: 1) aufweist, oder eines Globin-Proteolysats, welches das Peptid enthält, zur Herstellung einer Nahrungsmittelzusammensetzung zur Vorbeugung oder Behandlung einer Krankheit, die aus Hyperglykämie resultiert.

2. Verwendung eines Peptids, das die Sequenz Val-Val-Tyr-Pro (SEQ-ID Nr.: 1) aufweist, oder eines Globin-Proteolysats, welches das Peptid enthält, zur Herstellung einer Futtermittelzusammensetzung zur Vorbeugung oder Behandlung einer Krankheit, die aus Hyperglykämie resultiert.

3. Verwendung eines Peptids, das die Sequenz Val-Val-Tyr-Pro (SEQ-ID Nr.: 1) aufweist, oder eines Globin-Proteolysats, welches das Peptid enthält, zur Herstellung eines antidiabetischen Mittels.

4. Verwendung eines Peptids, das die Sequenz Val-Val-Tyr-Pro (SEQ-ID Nr.: 1) aufweist, oder eines Globin-Proteolysats, welches das Peptid enthält, zur Herstellung eines Nahrungsmittels für eine festgelegte Gesundheitsanwendung, das eine Funktion, einen Anstieg des Blutzuckers zu verhindern, oder eine Funktion, die Insulinsekretion zu steigern, aufweist und mit Blick auf eine solche Funktion verwendet wird.

5. Verwendung eines Peptids, das die Sequenz Val-Val-Tyr-Pro (SEQ-ID Nr. 1) aufweist, oder eines Globin-Proteolysats, welches das Peptid enthält, zur Herstellung eines Futtermittels für eine festgelegte Gesundheitsanwendung, das eine Funktion, einen Anstieg des Blutzuckers zu verhindern, oder eine Funkion, die Insulinsekretion zu steigern, aufweist und mit Blick auf eine solche Funktion verwendet wird.

6. Peptid, das die Sequenz Val-Val-Tyr-Pro (SEQ-ID Nr.:1) aufweist, oder ein Globin-Proteolysat, welches das Peptid enthält, zur Verwendung in der Vorbeugung oder Behandlung einer Krankheit, die aus einem hyperglykämischen Zustand, verursacht durch die Schwächung oder Abwesenheit der Funktion von Insulin, resultiert.

7. Peptid, das die Sequenz Val-Val-Tyr-Pro (SEQ-ID Nr.: 1) aufweist, oder ein Globin-Proteolysat, welches das Peptid enthält, zur Verwendung in der Vorbeugung oder Behandlung einer Krankheit, die aus einem hyperglykämischen Zustand, verursacht durch die Schwächung oder die Abwesenheit der Wirkung von Insulin, resultiert, nach Anspruch 6, wobei die Krankheit Diabetes oder eine diabetische Komplikation ist.

## Revendications

1. Utilisation d'un peptide possédant la séquence Val-Val-Tyr-Pro (SEQ ID NO: 1) ou d'un protéolysat de globine contenant le peptide pour préparer une composition alimentaire pour prévenir ou pour traiter une maladie résultant d'une hyperglycémie.

2. Utilisation d'un peptide possédant la séquence Val-Val-Tyr-Pro (SEQ ID NO: 1) ou d'un protéolysat de globine contenant le peptide pour préparer une composition d'aliments pour animaux pour prévenir ou pour traiter une maladie résultant d'une hyperglycémie.

3. Utilisation d'un peptide possédant la séquence Val-Val-Tyr-Pro (SEQ ID NO: 1) ou d'un protéolysat de globine contenant le peptide pour préparer un agent antidiabétique.

4. Utilisation d'un peptide possédant la séquence Val-Val-Tyr-Pro (SEQ ID NO: 1) ou d'un protéolysat de globine contenant le peptide pour préparer une denrée alimentaire destinée à une utilisation spécifiée concernant la santé, qui possède une fonction d'inhibition d'une augmentation de la glycémie ou une fonction d'augmentation de la sécrétion d'insuline et qui est utilisée en tenant compte de cette fonction.

5. Utilisation d'un peptide possédant la séquence Val-Val-Tyr-Pro (SEQ ID NO: 1) ou d'un protéolysat de globine contenant le peptide pour préparer un aliment pour animaux destiné à une utilisation spécifiée concernant la santé, qui possède une fonction d'inhibition d'une augmentation de la glycémie ou une fonction d'augmentation de la sécrétion d'insuline et qui est utilisée en tenant compte de cette fonction.

6. Peptide possédant la séquence Val-Val-Tyr-Pro (SEQ ID NO: 1) ou d'un protéolysat de globine contenant le peptide à utiliser dans la prévention ou dans le traitement d'une affection provoquée par un affaiblissement ou une carence de l'action de l'insuline.

7. Peptide possédant la séquence Val-Val-Tyr-Pro (SEQ ID NO: 1) ou d'un protéolysat de globine contenant le peptide à utiliser dans la prévention ou dans le traitement d'une affection provoquée par l'affaiblissement ou le manque de l'action de l'insuline selon la revendication 6, dans lequel la maladie est le diabète ou une complication diabétique.


### SEQUENCE LISTING

<110> MG PHARMA Inc.
   <120> Protein hydrolysate with antidiabetic effect
   <130> P05-98
   <150> US 60/627843
   <151> 2004-11-15
   <160> 1
<210> 1
   <211> 4
   <212> PRT
   <400> 1
   Val Val Tyr Pro
    1           4